# FASCICULE DE BREVET EUROPEEN

(11) **EP 1 115 399 B1**
(45) Date de publication et mention de la délivrance du brevet: **02.03.2005**
(21) Numéro de dépôt: 99929432.5
(22) Date de dépôt: 08.07.1999
(51) Int. Cl.: A61K 31/405, A61K 31/415, A61K 31/44, A61P 13/12

(54) **UTILISATION D'UNE COMPOSITION PHARMACEUTIQUE CONTENANT, EN ASSOCIATION, UN ANTAGONISTE DES RECEPTEURS AT1 DE L'ANGIOTENSINE II ET L'INDOMETHACINE POUR LA FABRICATION D'UN MEDICAMENT POUR TRAITER LES GLOMERULONEPHRITES CHRONIQUES**
VERWENDUNG EINER ZUSAMMENSETZUNG ENTHALTEND EINE ASSOZIATION EINES ANGIOTENSIN II AT1 REZEPTOR ANTAGONISTS UND INDOMETHACIN ZUR HERSTELLUNG EINES ARZNEIMITTELS ZUR BEHANDLUNG VON KRONISCHEN GLOMERULONEPHRITEN
USE OF A PHARMACEUTICAL COMPOSITION CONTAINING, IN COMBINATION, AN ANTAGONIST OF THE ANGIOTENSIN II AT 1? RECEPTORS AND INDOMETHACIN FOR MAKING A MEDICINE TREATING CHRONIC GLOMERULONEPHRITIS

(30) Priorité: 10.07.1998 FR 9808976
(43) Date de publication de la demande: 18.07.2001
(73) Titulaire: Sanofi-Aventis, 75013 Paris (FR)
(72) Inventeur: BROUARD, Rémi, F-75006 Paris (FR); REMUZZI, Giuseppe, I-24126 Bergamo (IT)
(74) Mandataire: Thouret-Lemaitre, Elisabeth
(86) Numéro de dépôt international: PCT/FR1999/001650
(87) Numéro de publication internationale: WO 2000/002556

(56) Documents cités:
- FRICKER ET AL: "effect of indomethacin on the renal response to the angiotensin II receptor antagonist valsartan in healthy subjects" J. OF HE AMERICAN SOCIETY OF NEPHROLOGY, vol. 9, septembre 1997 (1997-09), page 315a XP002122398
- PERICO ET AL: "the antiproteinuric effect of angiotensin antagonism in human IGA nephropathy is potentiated by indomethacin" J. AM. SOC. NEPHROL., vol. 9, no. 12, décembre 1998 (1998-12), pages 2308-2317, XP002122399
- FRICKER ET AL: "effect of indomethacin on the renal response to angiotensin II receptor blockade in healthy subjects" KIDNEY INTERNATIONAL, vol. 54, no. 6, 20 novembre 1998 (1998-11-20), pages 2089-2097, XP002122400
- OLSEN ET AL: "blood pressure and renal effects of indomethacin during angiotensin II receptor blockade with losartan" EUR. HEART J., vol. 19, août 1998 (1998-08), page 595 XP002122401

## Description

La présente invention a pour objet l'utilisation d'une composition pharmaceutique contenant en association l'irbesartan et l'indométhacine, Cette composition est particulièrement utile dans le traitement des glomérulonéphrites chroniques.

La possibilité d'associer un inhibiteur des récepteurs AT₁ de l'angiotensine II avec un antiinflammatoire non stéroïdien est décrite dans différents brevets ou demandes de brevets : EP-A-532410, US-5270317, EP-B-253310.

L'utilisation d'un inhibiteur des récepteurs AT₁ de l'angiotensine II est revendiquée dans plusieurs brevets ou demandes de brevets pour le traitement des troubles rénaux induits par l'administration d'antiinflammatoires non stéroïdiens : WO-9100281, US-5254546, EP-B-479903, US-5354867, WO-94-28896, US-5219856, EP-A-574174, US-5693633, EP-A-573271.

Par ailleurs, on trouve dans la littérature des études consacrées aux effets de l'association de certains antiinflammatoires non stéroïdiens avec, soit un antagoniste des récepteurs AT₁ de l'angiotensine II, soit un inhibiteur de l'enzyme de conversion.

Chez le rat spontanément hypertendu (rat SHR), l'indométhacine n'affecte pas la pression sanguine, mais il diminue l'effet antihypertenseur du losartan ou du ramipril (V. Cachofeiro : Hypertension, 1995, 26 (2), 236-243).

Chez des patients atteints d'insuffisance cardiaque, on a constaté que le losartan comme l'énalapril améliorent la consommation d'oxygène pendant l'exercice, et que l'aspirine s'oppose à cet effet s'il est dû à l'énalapril, mais ne s'y oppose pas s'il est dû au losartan (M. Guazzi : Am. J. Cardiol, 1997, 80 (12), 1572-1576).

Chez des patients souffrant d'insuffisance cardiaque, une administration d'aspirine concomitante au traitement par le losartan n'affecte pas les effets hémodynamiques du losartan (M.J. Frey : J. Am. Coll. Cardiol., 1996, 27 (2), suppl. A, 228A).

Par ailleurs, il est déconseillé d'utiliser un antagoniste des récepteurs AT₁ de l'angiotensine II en même temps qu'un antiinflammatoire non stéroïdien, à cause des effets hyperkalémiques de ces derniers (Y. Dimitrov : Presse Méd., 1997, 26 (40), 1981-1986).

Une publication de A.J. Fricker et al. dans J. Am. Soc. Nephrol., 1997 (9), 315a, décrit l'administration à des sujets sains du valsartan associé à l'indométhacine et les effets de cette association sur la vasodilatation rénale.

L'effet d'un ACE inhibiteur, le ramipril, sur la diminution du taux de filtration glomérulaire a été étudié par l'essai clinique REIN chez des patients atteints de maladie rénale protéinurique non diabétique (The Gisen Group : Lancet, 1997; 349, 1857-1863). On a montré que les inhibiteurs des récepteurs AT₁ de l'angiotensine II réduisent la protéinurie chez l'animal atteint de dysfonctionnements rénaux (G. Remuzzi *et al*. : Exp. Nephrol., 1996, 4, 19-25). Le losartan et l'énapril ont le même effet sur la diminution de l'excrétion des protéines urinaires (R.T. Gansevoort *et al*. : Kidney Int., 1993, 44, 579-584) . On sait depuis longtemps que les antiinflammatoires non stéroïdiens, et en particulier l'indométhacine, diminuent la protéinurie chez des patients présentant un syndrome néphrotique (A. Fieschi *et al*. : Prog. Med. Napoli, 1955, 9, 257 - A.J. Donker *et al*. : Nephron, 1978, 22, 374-381 - H. Golbetz *et al.* : Am. J. Physiol, 1989, 256, F44-F51).

Par ailleurs, l'additivité des effets antiprotéinuriques d'un inhibiteur de l'enzyme de conversion et d'un antiinflammatoire non stéroïdien a été montrée (J.E. Heeg, Clinical Science, 1991, 81, 367-372). Selon cette étude, on voit que l'excrétion des protéines urinaires chute d'environ 80 % après 1 mois de traitement par l'indométhacine associé au lisonipril ; parallèlement, on constate que le taux de filtration glomérulaire (TFG) diminue significativement de 25 % alors qu'il reste pratiquement stable au cours du traitement par le lisonipil seul.

On a maintenant trouvé, de façon surprenante, qu'il est intéressant d'associer l'irbesartan et l'indométhacine et que cette association peut être utile dans le traitement des glomérulonéphrites chroniques.

On a maintenant trouvé que l'association de l'indométhacine avec l'irbesartan, soit tel quel, soit sous forme polymorphe, soit sous forme d'un de ses sels ou solvats, présente une efficacité inattendue et un avantage particulier.

Ainsi, une telle association peut permettre de diminuer les doses de chacun des composés de l'association tout en conservant la même réponse pharmacologique que celle obtenue lorsqu'ils sont utilisés seuls.

L'association selon la présente invention peut être utilisée notamment pour le traitement des glomérulonéphrites chroniques. Par glomérulonéphrite chronique, on entend en particulier les glomérulonéphrites chroniques à forme mésangiales ou à IgA ou glomérulonéphrites mésangioprolifératives ou maladie de Berger.

L'association selon l'invention comprenant l'irbesartan et l'indométhacine a été étudiée chez l'homme.

Des patients atteints de glomérulonéphrite chronique sont traités pendant 28 jours par 100 mg par jour d'irbesartan, les 3 jours suivants ils reçoivent, en plus des 100 mg d'irbesartan, 75 mg d'indométhacine 2 fois par jour.

Pour chaque patient, on mesure l'excrétion en protéines pendant 24 heures et on étudie la clairance rénale pour évaluer le taux de filtration glomérulaire (TFG), le flux rénal plasmatique (FRP) et la clairance fractionnée de l'albumine.

7 jours avant le début du traitement (J-7), le premier jour du traitement J1) et après 2 semaines (J14), 4 semaines (J28) puis 31 jours de traitement.

La concentration totale des protéines dans les urines est mesurée automatiquement (Synchron® CX5, Beckman, Fulenton, Californie, Etats-Unis).

Les résultats sont rapportés dans les tableaux 1 et 2 suivants :

**Tableau 1 -**

| Excrétion des protéines urinaires : valeur de base (J-7), avant la première administration (J1), après 14 jours (J14) puis 28 jours de traitement par l'irbesartan (J28) et après 3 jours de traitement combiné avec l'irbesartan + l'indométhacine (J31) | | | | | | |
|---|---|---|---|---|---|---|
| | | J-7 Valeur de base | J1 | J14 | J28 irbesartan | J31 irbesartan + indométhacine |
| Protéines urinaires | g/24H | 2,48±2,02 | 2,38±1,78 | 1,71±1,61 | 1,54±1,46* | 0,57±0,43* |
| Valeur moyenne ± (DS) | | | | | | |

| | | | | | | |
|---|---|---|---|---|---|---|
| * p < 0,05 vs J1 | | | | | | |

**Tableau 2 -**

| Paramètres systémiques et paramètres fonctionnels rénaux : valeur de base (J-7), pendant la première administration d'irbesartan (J1), après 28 jours de traitement par l'irbesartan (J28) et après 3 jours de traitement combiné avec l'indométhacine (J31) | | | | | |
|---|---|---|---|---|---|
| | | J-7 Valeur de base | J1 | J28 Irbesartan | J31 Irbesartan + indométhacine |
| TFG | ml/min/1,73 sqm | 54±+15 | 56±1 8 | 55±11 | 54±11 |
| FRP | ml/min/1,73 sqm | 430±110 | 478±153 | 549±117** | 518±143 |
| FF | % | 13± 3 | 12±3 | 10±1 | 11±2 |
| Clairance | mini-maxi | (3-217) | (4-217) | (6-96) | (2-91) |
| Albumine fractionnée | x 10-5 | 34,6 | 30,3 | 24,5 | 4,5⁺⁺ |

| | | | | | |
|---|---|---|---|---|---|
| FF : fraction de filtration | | | | | |
| TFG : taux de filtration glomérulaire | | | | | |
| FRP : flux rénal plasmatique Valeur moyenne (± DS) ou (minimun-maximum) | | | | | |
| ** p < 0,01 vs J-7 et J1 | | | | | |
| ++ p < 0,01 vs J28 | | | | | |

D'après le tableau 1, on voit que le traitement par l'irbesartan induit une réduction significative du taux d'excrétion des protéines urinaires apparente dès le jour 14 et encore augmentée au jour 28. La réduction de la protéinurie est statistiquement significative après 28 jours de traitement. De plus, l'indométhacine induit une réduction supplémentaire significative de cette protéinurie. Pour la clairance de l'albumine fractionnée, on observe que le traitement par l'irbesartan diminue seulement numériquement cette clairance, alors que le traitement avec l'indométhacine et l'irbesartan réduit significativement cette clairance.

D'après le Tableau 2 TFG et FF ne changent pas pour les patients ayant reçu le traitement par l'irbesartan seul ou associé à l'indométhacine alors que FRP augmente.

Ainsi, on constate qu'après 3 jours de traitement par l'association de l'indométhacine et de l'irbesartan, le taux d'excrétion des protéines urinaires a baissé de 77 % et que, pendant le même temps, le taux de filtration glomérulaire est inchangé.

Ainsi, on a constaté que le traitement par l'association de l'irbesartan et de l'indométhacine a permis de potentialiser les effets de l'irbesartan en diminuant à la fois l'excrétion des protéines urinaires et la clairance de l'albumine fractionnée, tandis que les autres fonctions rénales sont inchangées.

Pour son utilisation en tant que médicament, l'association de l'indométhacine et de l'irbesartan doit être formulée en composition pharmaceutique. Ladite composition est généralement formulée en unités de dosage.

La présente invention concerne des compositions pharmaceutiques comprenant l'indométhacine et l'irbesartan en association avec au moins un excipient pharmaceutique.

Dans les compositions pharmaceutiques de la présente invention pour l'administration par voie orale, sublinguale, inhalée, sous-cutanée, intramusculaire, intraveineuse, transdermique, locale ou rectale, les principes actifs de l'association peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topique, les implants, les formes d'administration sous-cutanée, intramusculaire, intraveineuse, intranasale ou intraoculaire et les formes d'administration rectale.

Lorsque l'on prépare une composition solide sous forme de comprimés, on ajoute aux principes actifs de l'association, micronisés ou non, un véhicule pharmaceutique qui peut être composé de diluants comme par exemple le lactose, la cellulose microcristalline, l'amidon et des adjuvants de formulation comme des liants (polyvinylpyrrolidone, hydroxypropylméthylcellulose, etc...), des agents d'écoulement comme la silice, des lubrifiants comme le stéarate de magnésium, l'acide stéarique, le tribéhénate de glycérol, le stéarylfumarate de sodium.

Des agents mouillants ou tensioactifs tels que le laurylsulfate de sodium peuvent être ajoutés à la formulation.

Les comprimés peuvent être réalisés par différentes techniques, compression directe, granulation sèche, granulation humide, fusion à chaud.

Les comprimés peuvent être nus, dragéifiés (par du saccharose par exemple) ou enrobés avec divers polymères ou autres matières appropriées.

Les comprimés peuvent avoir une libération flash, retardée ou prolongée en réalisant des matrices polymériques ou en utilisant des polymères spécifiques au niveau du pelliculage.

On obtient une préparation en gélule par mélange des principes actifs avec des véhicules pharmaceutiques secs (simple mélange ou granulation sèche, granulation humide, fusion à chaud), liquides ou semi-solides.

Les gélules peuvent être molles ou dures, pelliculées ou non de manière à avoir une activité flash, prolongée ou retardée (par exemple par une forme entérique).

Une préparation sous forme de sirop ou d'élixir peut contenir les principes actifs conjointement avec un édulcorant, acalorique de préférence, du méthylparaben et du propylparaben comme antiseptique, ainsi qu'un agent donnant du goût et un colorant approprié.

Une préparation sous forme de sirop ou d'élixir peut contenir les principes actifs conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir les principes actifs en mélange avec des agents de dispersion, des agents mouillants ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol ou le butylèneglycol.

Ainsi, pour préparer une solution aqueuse injectable par voie intraveineuse on peut utiliser un cosolvant comme par exemple un alcool tel que l'éthanol ou un glycol tel que le polyéthylèneglycol ou le propylèneglycol, et un tensioactif hydrophile tel que le Tween® 80. Pour préparer une solution huileuse injectable par voie intramusculaire, on peut solubiliser les principes actifs par un triglycéride ou un ester de glycérol.

Pour l'administration locale on peut utiliser des crèmes, des pommades, des gels, des collyres.

Pour l'administration transdermique, on peut utiliser des patches sous forme multilaminée ou à réservoir dans lequel les principes actifs peuvent être en solution alcoolique.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane ou tout autre gaz propulseur biologiquement compatible ; on peut également utiliser un système contenant les principes actifs seul ou associé à un excipient, sous forme de poudre.

Les principes actifs peuvent être également présentés sous forme de complexe avec une cyclodextrine, par exemple, α, β, γ-cyclodextrine, 2-hydroxypropyl-β-cyclodextrine, méthyl-β-cyclodextrine.

Les principes actifs peuvent être formulés également sous forme de microcapsules ou microsphères, éventuellement avec un ou plusieurs supports ou additifs.

Parmi les formes à libération prolongée utiles dans le cas de traitements chroniques, on peut utiliser les implants. Ceux-ci peuvent être préparés sous forme de suspension huileuse ou sous forme de suspension de microsphères dans un milieu isotonique.

Dans chaque unité de dosage les principe actifs de l'association sont présents dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 10 à 150 mg d'indométhacine, de préférence de 25 à 100 mg, et de 0,5 à 500 mg d'irbesartan, de préférence de 1 à 300 mg, ladite unité de dosage devant être administrée une à quatre fois par jour.

Selon un autre de ses aspects, la présente invention concerne l'utilisation de l'association selon l'invention pour la préparation de médicaments destinés à traiter les affections de la sphère rénale chez l'homme et chez l'animal.

Tout particulièrement, la composition pharmaceutique selon l'invention est utile pour la fabrication d'un médicament pour traiter les glomérulonéphrites chroniques.

Les compositions de la présente invention peuvent contenir, à côté de l'association selon l'invention, d'autres principes actifs qui peuvent être utiles dans le traitement des troubles ou maladies indiquées ci-dessus.

Ainsi, selon la présente invention, on peut préparer des compositions pharmaceutiques contenant l'association selon l'invention associée à un autre composé agissant sur le système rénine-angiotensine tel qu'un inhibiteur de l'enzyme de conversion ou un inhibiteur de la rénine. On peut également associer l'association selon l'invention, par exemple, avec un vasodilatateur périphérique, un inhibiteur calcique, un béta-bloquant, un alpha-1-bloquant ou un diurétique.

Ces associations permettront de renforcer les activités thérapeutiques de l'association selon l'invention.

| EXEMPLE 1 Gélule | |
|---|---|
| Indométhacine | 50,00 mg |
| Irbesartan | 150,00 mg |
| Lactose monohydrate | 252,35 mg |
| Amidon de maïs modifié | 57,77 mg |
| Silice colloïdale anhydre | 2,13 mg |
| Stéarate de magnésium | 4,25 mg |
| Talc | 8,50 mg |
| Pour une gélule blanc opaque n° 0 remplie à | 425 mg |

| EXEMPLE 2 Gélule | |
|---|---|
| Indométhacine | 100,00 mg |
| Irbesartan | 300,00 mg |
| Lactose monohydrate | 138,60 mg |
| Amidon de maïs modifié | 46,52 mg |
| Silice colloïdale anhydre | 2,13 mg |
| Stéarate de magnésium | 4,25 mg |
| Talc | 8,50 mg |
| Pour une gélule blanc opaque n° 0 remplie à | 600 mg |

| EXEMPLE 3 Gélule | |
|---|---|
| Indométhacine | 50,00 mg |
| Irbesartan | 300,00 mg |
| Lactose monohydrate | 188,60 mg |
| Amidon de maïs modifié | 46,52 mg |
| Silice colloïdale anhydre | 2,13 mg |
| Stéarate de magnésium | 4,25 mg |
| Talc | 8,50 mg |
| Pour une gélule blanc opaque n° 0 remplie à | 600 mg |

## Revendications

1. Composition pharmaceutique contenant en association l'indométhacine et l'irbesartan, soit tel quel, soit sous forme polymorphe, soit sous forme d'un de ses sel s ou solvats.

2. Composition pharmaceutique selon la revendication 1 dans laquelle les principes actifs de l'association sont mélangés à au moins un excipient pharmaceutique.

3. Composition pharmaceutique selon la revendication 2 sous forme d'unité de dosage.

4. Composition pharmaceutique selon la revendication 3 contenant 10 à 150 mg d'indométhacine et 10 à 300 mg d'irbesartan.

5. Composition pharmaceutique selon la revendication 4 contenant de 25 à 100 mg d'indométhacine et 50 à 250 mg d'irbesartan.

6. Utilisation d'une composition pharmaceutique selon l'une quelconque des revendications 1 à 5 pour la fabrication de médicaments utiles pour traiter les glomérulonéphrites chroniques.

## Patentansprüche

1. Pharmazeutische Zubereitung enthaltend in Kombination Indomethacin und Irbesartan entweder als solche oder in polymorpher Form oder in Form eines ihrer Salze oder Solvate.

2. Pharmazeutische Zubereitung nach Anspruch 1, worin die kombinierten Wirkstoffe mit mindestens einem pharmazeutischen Trägermaterial vermischt sind.

3. Pharmazeutische Zubereitung nach Anspruch 2 in Form einer Dosiseinheit.

4. Pharmazeutische Zubereitung nach Anspruch 3, enthaltend 10 bis 150 mg Indomethacin und 10 bis 300 mg Irbesartan.

5. Pharmazeutische Zubereitung nach Anspruch 4, enthaltend 25 bis 100 mg Indomethacin und 50 bis 250 mg Irbesartan.

6. Verwendung einer pharmazeutischen Zubereitung nach einem der Ansprüche 1 bis 5 für die Herstellung von Arzneimitteln, die nützlich sind zur Behandlung von chronischer Glomerulonephritis.

## Claims

1. Pharmaceutical composition containing, in combination, indomethacin and irbesartan, either as it is, or in polymorphic form, or in the form of one of its salts or solvates.

2. Pharmaceutical composition according to Claim 1, in which the active principles of the combination are mixed with at least one pharmaceutical excipient.

3. Pharmaceutical composition according to Claim 2, in dosage unit form.

4. Pharmaceutical composition according to Claim 3, containing 10 to 150 mg of indomethacin and 10 to 300 mg of irbesartan.

5. Pharmaceutical composition according to Claim 4, containing from 25 to 100 mg of indomethacin and 50 to 250 mg of irbesartan.

6. Use of a pharmaceutical composition according to any one of Claims 1 to 5, for producing medicinal products that are useful for treating chronic glomerulonephritis.
